# EUROPEAN PATENT APPLICATION

(11) **EP 3 772 353 A1**
(43) Date of publication of application: **10.02.2021**
(21) Application number: 19190486.1
(22) Date of filing: 07.08.2019
(51) Int. Cl.: A61H 7/00, A61H 23/02

(54) **APPARATUS AND METHOD FOR TACTILE STIMULATION OF HUMAN SKIN**

(71) Applicant: Di Lernia, Daniele, 20017 Rho (MI) (IT)
(72) Inventor: Di Lernia, Daniele, 20017 Rho (MI) (IT)
(74) Representative: De Bortoli, Tiziano

(57) **Abstract**

It is disclosed an apparatus (100) for tactile stimulation of C-Tactile afferents comprising a casing (10) configured to be positioned onto a user's skin and at least one tactile stimulation element (21) carried by the casing (10) and operated by a motor (30) at a controlled velocity for generating at least one tactile stimulation. The apparatus (100) further comprises an electronic control unit (40) and at least one pressure sensor (50) in electrical communication with the electronic control unit (40) to control the pressure force exerted by the tactile stimulation element (21) onto the user's skin by adjusting the position of the tactile stimulation element (21) with respect to the user's skin, based on a feedback signal transmitted by said at least one pressure sensor (50). A method of generating at least one tactile stimulation of C-Tactile afferents carried out by using the aforesaid apparatus (100) is also disclosed.

## Description

### Technical field of the invention

The present invention relates, in general, to the technical field of tactile stimulation. In particular, the present invention relates to an apparatus and method for providing non-invasive interoceptive tactile stimulation of human skin for treating pathological conditions, such as, for example, pain both acute and chronic, anxiety, chronic stress, addictions, depression, post-traumatic stress disorder (PTSD) and emotional dysregulations in other pathologies such as autism, attention deficit hyperactivity disorder (ADHD) etc.

### Background of the invention

In highly social species, across the lifespan, touch plays a central role in the formation and maintenance of relationships. Between an infant and caregiver, within primate hierarchies and in romantic relationships, tactile interactions are rewarding, buffer physiological and psychological responses to stress and ultimately enhance well-being. For example, parental touch is a key regulator of an infant's physiological and behavioural arousal and, in adults, physical contact has been shown to modulate physiological responses to an acute stressor, to increase the autonomic wellbeing, and to reduce the perception of pain.

Several types of tactile perceptions exist, and they are processed by different areas of our brain, providing different psycho-physiological effects upon our body. Nonetheless, despite this centrality of touch to our bodily perception and well-being, to date, little attention has been paid to the neurophysiological basis of its beneficial effects. Among different types of touch, the interoceptive touch is a very specific secondary type of tactile perception that is processed by the interoceptive system and demonstrated a broad range of psychophysiological beneficial effects.

The study of the interoceptive system represents an emerging and promising topic in neuroscience, due to the meaning that interoceptive information has to the functioning of the organism. Interoceptive perceptions can be defined as the sense of the physiological status of the entire organism and they encompass a broad range of relevant biological functions that serve conscious and unconscious processes.

The central component of the interoceptive system is the anterior insular cortex (AIC) that receives information through a vast network of small unmyelinated fibers connected to the Lamina I spinothalamocortical pathway. These unmyelinated fibers compose a poly-modal afferent system that innervates the entire organism and report a wide range of inputs such as: hunger, thirst, pain, itch, temperature, muscle contraction, hormonal and immune activity, cardiorespiratory function, and a specific kind of touch, with unique neuro-physiological effects.

The inputs are processed in the interoceptive matrix according to an explicit lateralization of the cerebral cortex. Specifically, the left and right insula of the cerebral cortex are usually coactive in the interoceptive system; nonetheless parasympathetic inputs are preferentially processed by the left insula, while sympathetic inputs are usually processed by the right insula.

Recent evidence identified interoceptive alterations in a broad range of clinical conditions, such as chronic pain, eating disorders, anxiety, depression, addictions, post traumatic stress disorders, insomnia, and several other conditions. This evidence suggests that a device able to stimulate of the interoceptive system could provide a therapeutic solution for such conditions.

As stated above, the interoceptive system is composed by a poly-modal afferent network of C-fibers. These fibers are unique and are able to collect a broad range of information. Interestingly, the C-fibers differentiate in free tactile arborizations on the superficial layer of the derma, creating a secondary touch system that is specifically connected to and processed by the interoceptive cortical areas. C-Tactile afferents (CTs) are unmyelinated low threshold mechanoreceptors innervating the skin of mammals and they are uniquely found in not-glabrous skin.

In humans, their response characteristics have been elegantly mapped using single-unit microneurography recordings, establishing that C-Tactile afferents only respond to a precise low force and velocity skin temperature stimulus moving across their receptive field.

In particular, it has been found that C-Tactile afferents show the highest firing frequency to light stroking with a pressure force equal to or lower than 2.5 mN and within a stroking velocity between 1 and 10 cm/s, with an optimal velocity of 3 cm/s. Moreover, C-Tactile afferents exhibit a tendency to fatigue and a specific after-discharge pattern with a delayed acceleration effect.

Intriguingly, several clinical conditions demonstrated alterations in perceptions that were connected to C-Tactile afferents, suggesting an implication for interoceptive touch also in psychopathological functioning. As a further matter, human and animal models indicated that interoceptive touch can reduce and modulate pain perception, anxiety, and chronic stress.

The above considerations suggest a promising role of interoceptive stimulation in different fields, from clinical applications to sensing technology and assessment; nevertheless, the same considerations underline the need for apparatuses that can supply interoceptive tactile stimulation, both for applied research and clinical treatments.

Such apparatuses actually exist configured to supply interoceptive tactile stimulation. However, the known apparatuses present some drawbacks.

Firstly, they are unable to automatically regulate the tactile stimulation based on the physical characteristics of the user's skin, and thus the stimulation treatment must be carried out by specialized personnel, in order to stimulate the C-Tactile afferents with the above indicated optimal stroking force and velocity values. Interoceptive tactile afferents respond exclusively to a narrow set of parameters that must be constantly monitored and tuned to ensure a proper activation of the interoceptive system and its following beneficial effects on the organism.

The physical characteristics of the user's skin, such as for example the thickness of the fat layer, the dimension of the body section to stimulate, the specific body morphological structure can interfere with the stimulation parameters, reducing or even cancelling the effects of the interoceptive tactile stimulation, which must be constantly maintained within strict parameters to express its therapeutic effects.

Second, the known apparatuses for interoceptive tactile stimulation are typically connected to a fixed setup, limiting the portability of the apparatuses in different locations, such as hospitals, laboratories and the like, and the possibility to apply stimulation to different body parts of a user. A known stimulation apparatus is described, for example, in Di Lernia, D., P. Cipresso, E. Pedroli and G. Riva (2018). "Toward an Embodied Medicine: A Portable Device with Programmable Interoceptive Stimulation for Heart Rate Variability Enhancement". Sensors (Basel) 18(8).

The main object of the present invention is therefore to provide an apparatus and a method for tactile stimulation of C-Tactile afferents configured to overcome or at least reduce the drawbacks above mentioned with reference to the known tactile stimulation apparatuses.

More specifically, the main object of the present invention is to provide an apparatus for tactile stimulation of C-Tactile afferents configured to automatically regulate the tactile stimulation based on the physical characteristics of the user's skin, so as to stimulate the C-Tactile afferents with the above indicated optimal stroking force and velocity.

Another object of the present invention is to provide an apparatus for tactile stimulation of C-Tactile afferents configured to be portable in different locations, such as hospitals, laboratories and the like, and also configured to allow home treatment.

Another object of the present invention is to provide an apparatus for tactile stimulation of C-Tactile afferents configured to allow a plurality of apparatuses to be used for simultaneously stimulating different body parts of a user.

A further object of the present invention is to provide an apparatus for tactile stimulation of C-Tactile afferents which can be produced at competitive costs.

The above-mentioned objects, and other objects that will better appear in the following of the present description, are achieved by an apparatus and a method for tactile stimulation of C-Tactile afferents as defined in the attached independent claims 1 and 11. Preferred characteristics of the apparatus and method for tactile stimulation of C-Tactile afferents are set forth in the dependent claims 2-10 and 12-15, respectively.

In a first aspect thereof, the present invention relates to an apparatus for tactile stimulation of C-Tactile afferents comprising a casing configured to be positioned onto a user's skin and at least one tactile stimulation element carried by the casing and operated by a motor at a controlled velocity for generating at least one tactile stimulation.

The apparatus is characterized in that it further comprises an electronic control unit and at least one pressure sensor in electrical communication with the electronic control unit to control the pressure force exerted by the at least one tactile stimulation element onto the user's skin by adjusting the position of the tactile stimulation element with respect to the user's skin based on a feedback signal transmitted from said at least one pressure sensor.

Preferably, the at least one tactile stimulation element is controlled to exert onto the user's skin a pressure force matching a value requested by the C-Tactile afferents.

In a second aspect thereof, the present invention relates to a method of generating at least one tactile stimulation comprising the steps of:
- operating at least one tactile stimulation element at a controlled velocity to generate at least one tactile stimulation;
- adjusting the position of the at least one tactile stimulation element with respect to the user's skin to control the pressure force exerted by the at least one tactile stimulation element onto the user's skin, based on a feedback signal transmitted by at least one pressure sensor.

Preferably, the at least one tactile stimulation element is lowered/raised to exert onto the user's skin a pressure force matching a value requested by the C-Tactile afferents.

Due to the above combination of features, in particular due to the presence of the one or more pressure sensors, the apparatus and the method defined above are able to automatically maintain precise stimulation parameters of the C-Tactile afferents, in particular the stroking force, regardless of the physical characteristics of the user's body.

Therefore, the apparatus according to the present invention advantageously allows the C-Tactile afferents to be stimulated with the optimal stroking force and velocity values, without the intervention of and the specialized personnel.

Advantageously, the apparatus for tactile stimulation of C-Tactile afferents of the present invention is so sized to be portable in different locations, for example at the user's home for home treatment, and to allow a plurality of apparatuses to be simultaneously used to stimulate different body parts of a user.

In a further aspect thereof, the present invention relates to the use of the apparatus and of the method defined above for producing pain analgesia and stress relief.

Hereafter, in the present description, and in the claims, the expression "tactile stimulation" is referred to stimulation of C-Tactile afferents or interoceptive tactile stimulation.

### Brief description of the drawings

Further features and advantages of the present invention will become more readily apparent from the following detailed description of a preferred embodiment of an apparatus and a method for generating at least one tactile stimulation, provided below, by way of non-limiting indication, with reference to the accompanying drawings. In the drawings:
- Figure 1 is a schematic top view of an apparatus for tactile stimulation according to a first embodiment of the present invention;
- Figure 2 is a schematic perspective view of the apparatus of Figure 1;
- Figure 3 is a schematic cross-sectional view of the apparatus of Figure 1, with the tactile stimulation element in the retracted or rest condition thereof;
- Figure 4 is a schematic cross-sectional view of the apparatus of Figure 1, with the tactile stimulation element in the extracted or operative condition thereof;
- Figure 5 schematically shows the way of interaction between the different components of the apparatus according to the present invention;
- Figure 6 is a schematic cross-sectional view of a second embodiment of the apparatus for tactile stimulation according to the present invention, with the tactile stimulation element in the retracted or rest condition thereof;
- Figure 7 is a schematic cross-sectional view of the apparatus of Figure 6, with the tactile stimulation element in the extracted or operative condition thereof;
- Figure 8 is an enlarged view of the particular circled with C in Figure 7; and
- Figure 9 is a flow chart, schematically showing the different steps of a method of tactile stimulation of C-Tactile afferents carried out with the apparatuses of Figures 1-8.

### Description of preferred embodiments of the invention

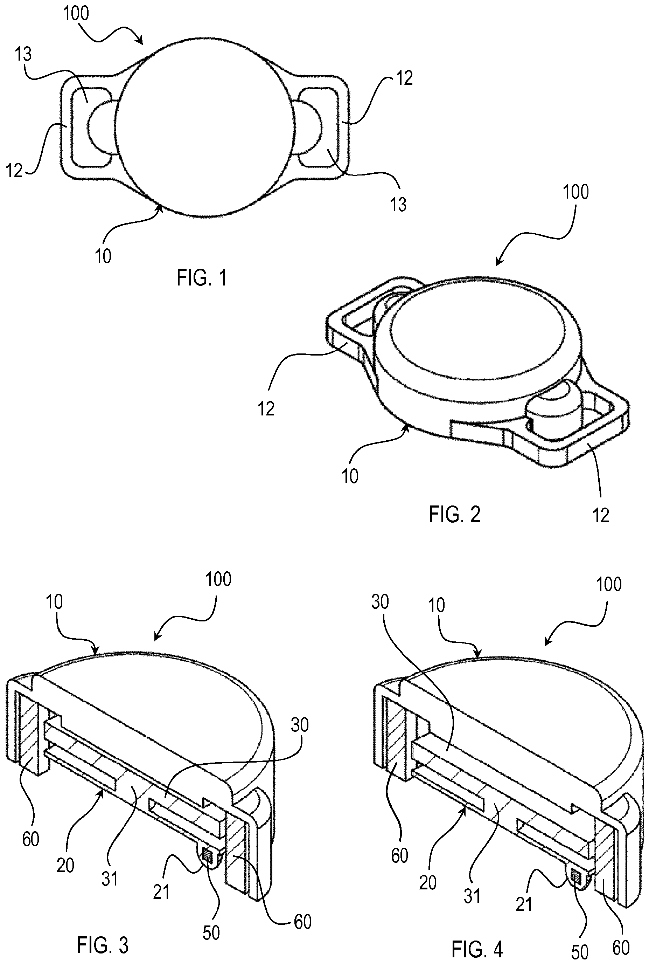

With reference to Figures 1 to 5, a tactile stimulation apparatus according to a first embodiment of the present invention is indicated, in general, with the reference numeral 100.

The apparatus 100 comprises a casing 10, which is configured to house the mechanical and electronical components of the apparatus, which will be described in detail below.

The casing 10 is preferably made of plastic material and is suitably dimensioned to be portable and easily positioned on the user's body part to be stimulated. For this purpose, the casing 10 comprises a pair of tabs 12, which laterally extend from the casing 10, opposite each other. Each tab 12 is provided with an aperture 13 through which a preferably elastic band (not shown) can pass to suitably position the apparatus onto the user's skin.

The apparatus 100 further comprises a tactile stimulation element 21 configured to stimulate C-Tactile afferents of the user's skin with predetermined stimulation parameters.

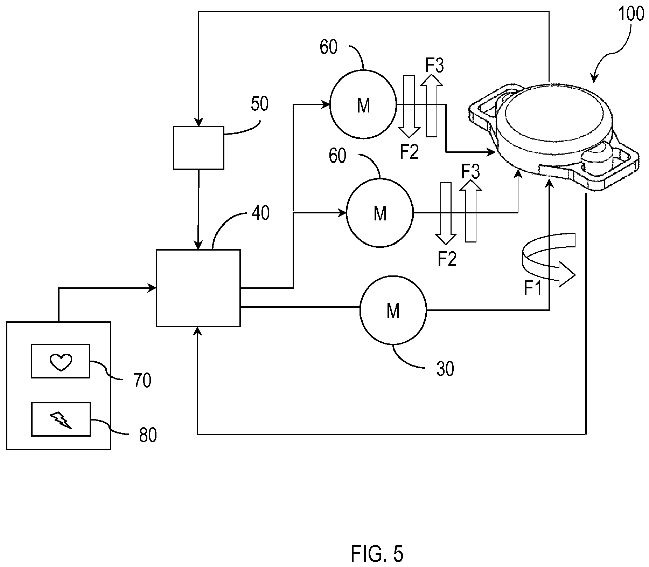

In the embodiment shown in Figures 1 to 4, the tactile stimulation element 21 projects downwards from a peripheral portion of a stimulation disk 20 supported by the casing 10, to contact, in use, the user's skin. Naturally, it is possible to provide a plurality of tactile stimulation elements 21, each downwardly projecting from the peripheral portion of the stimulation disk 20. The stimulation disk 20 has preferably a circumference of 10 cm and is connected to a main shaft 31 of a motor 30, which drives the stimulation disk 20, and the tactile stimulation element 21 downwardly projecting therefrom, to generate at least one interoceptive tactile stimulation. Preferably, the motor 30 is a rotary motor, for example a stepper/servo rotary motor, preferably a piezoelectric rotary motor, which moves, preferably rotates (see arrow F1 in Figure 5) the tactile stimulation element 21 at a controlled velocity for generating a circular stimulation pattern.

In a preferred embodiment thereof, the tactile stimulation element 21 carried by the stimulation disk 20 has an oval-shape. This preferred configuration of the tactile stimulation element 21 allows a circular stimulation pattern at a specific calibrated velocity thus helps avoiding C-Tactile afferents fatigue and adaptation. C-Tactile afferents tend to exhibit fatigue and adaptation after 5s of continuous stimulation. The circular stimulation patter provided by the tactile stimulation element 21 mounted on the stimulation disk 20, allows to constantly change the site of stimulation, ensuring that CT afferent patches are directly stimulated only for short period of time (< 5s) avoiding fatigue and habituation of the receptors.

As shown in greater detail in Figure 5, the apparatus 100 further comprises an electronic control unit 40, which is in electrical communication with the motor 30 to control the velocity of the tactile stimulation element 21, and thus the C-Tactile afferents stroking velocity. More specifically, and preferably, the motor 30 connected to the tactile stimulation element 21 is controlled by the electronic control unit 40 to produce an interoceptive tactile stimulation at a velocity between 1 cm/s to 10 cm/s, with optimal velocity set to 3 cm/s, velocities at which, how disclosed above, the CTs show the highest firing frequency.

The apparatus 100 further comprises at least one pressure sensor 50 configured to detect the pressure force exerted by the tactile stimulation element 21 onto the user's skin, during the operation of the apparatus.

As schematically illustrated in Figures 3 and 4, the apparatus 100 comprises a pressure sensor 50, which is embedded in the tactile stimulation element 21. Preferably, the pressure sensor 50 is in the form of micro load cell, pressure sensitive graphene polymers, or any other means able to detect the forces required by the interoceptive tactile stimulation. As shown in greater detail in Figure 5, the pressure sensor 50 is in electrical communication with the electronic control unit 40, which in turn, utilizes a feedback signal transmitted by the pressure sensor 50 to adjust the position of the tactile stimulation element 21 with respect to the user's skin. More specifically, the electronic control unit 40 operates a further motor 60, preferably a pair of motors 60, to adjusting the position of the tactile stimulation element 21 with respect to the user's skin. More specifically, the tactile stimulation element 21 is lowered (see arrow F2) or raised (see arrow F3) by the further motor(s) to produce a pressure force lower than 40 mN, preferably equal to about 2.5 mN. In fact, as disclosed above, C-Tactile afferents show the highest firing frequency to light stroking with a pressure force equal to about 2.5 mN.

With reference to Figure 5, the electronic control unit 40 of the apparatus 100 can be electrically connected with one or more biosensors 70, 80, for example - but not limited to - ECG electric sensors, galvanic skin response sensors, temperature sensors, plethysmographic sensors, respiratory band sensors, graphene haptic sensors, which are configured to detect a physiological data of the user, for example hear rate, heart rate variability, sympathetic and parasympathetic responses, stress level, oxygen saturation, and to transmit the detected data to the electronic control unit 40. The electronic control unit 40, in turn, uses the received physiological data to regulate the tactile stimulation according to the user's physiological response. These biosensors can be either embedded in the apparatus 100, or the apparatus can interface with external biosensors such as smart fitness bracelets, fitness bands and other devices, to collect physiological data and modulate the stimulation accordingly.

The apparatus 100 further comprises a battery (not shown), preferably of the rechargeable type, for supplying power to the motors 30 and 60, the pressure sensor 50, the biosensors when embedded in the apparatus, and the electronic control unit 40. The battery is also housed in the casing 10. The apparatus 100 can be equipped with a BLE (Bluetooth low energy) module connected to the electronic control unit 40. The BLE module allows wireless pairing with a smartphone, a personal computer, or any other electronic device that will host the user interface program.

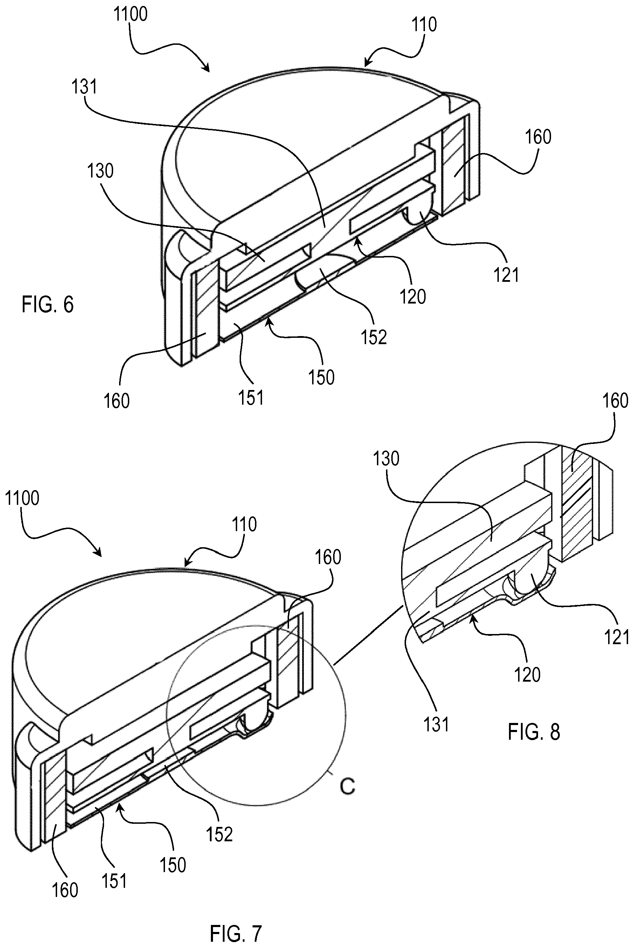

Figures 6 to 8 illustrate an apparatus for tactile stimulation according to a second embodiment of the present invention.

The apparatus, general designed with the reference numeral 1100, is similar to the apparatus 100 described above and illustrated in Figures 1-5, from which differs for the different configuration of the pressure sensor. Therefore, the apparatus 1100 comprises a casing 110 and a stimulation disk 120 supported by the casing 110 and provided with a tactile stimulation element 121 downwardly projecting from a peripheral portion of the stimulation disk 120 to contact, in use, the user's skin. A plurality of tactile stimulation elements 121 can be also provided, each projecting from the peripheral portion of the stimulation disk 120.

The stimulation disk 120, and the at least one tactile stimulation element 121 downwardly projecting therefrom, are operated, preferably rotated by a motor 130 for generating a circular stimulation pattern. The tactile stimulation element 121 is the same of the stimulation element 21 and therefore it is not further described.

The apparatus 1100 comprises an electronic control unit 140 in electrical communication with the motor 130 to control the velocity of the tactile stimulation element 121 and thus the C-Tactile afferents stroking velocity. The apparatus 1100 further comprises a pressure sensor 150, which unlike the pressure sensor 50 disclosed above is not embedded in the stimulation element, but is placed under the tactile stimulation element 121, so as to be interposed, in use, between the stimulation element 121 and the user's skin.

More specifically, the pressure sensor comprises a membrane 151 inside with a disk 152 made of graphene-composed polymer is housed. The membrane 151 is fixed to the casing 110 at its peripheral edge.

The membrane 151 is made of a flexible material and has a thickness lower than 1 mm. The disk 152 comprises electrical connections (not shown) to the electronic control unit 140, which, based on a feedback signal transmitted by the pressure sensor 150, operates a further motor 160, preferably a pair of motors 160, to adjust the position of the tactile stimulation element 121 with respect to the user's skin. More specifically, the tactile stimulation element 121 is lowered or raised by the further motor(s) 160 to produce on the C-Tactile afferents a stroking force lower than 40 mN, preferably equal to 2.5 mN.

The pressure sensor 150 made in the form of a graphene membrane advantageously allows to detect very small pressure values, typically used during the interoceptive tactile stimulation.

The apparatuses 100, 1100 according the first and second embodiments described above, further comprises a user interface (not shown), through which a user may select a set of stimulation parameters, more specifically stroking time, inter stimulus intervals, or pattern (frequency) of the stimulation. These stimulation parameters may be combined together thereby creating different types of interoceptive tactile stimulation.

In continuous stimulation, the apparatus 100, 1100, specifically the tactile stimulation element 21, 121, is controlled to provide a continuous interoceptive stimulus. The stroking time or duration of the stimulation may be stored in a memory, for example a flash memory, of the electronic control unit 40, 140; otherwise, the movement of the tactile stimulation element 21, 121 can be stopped at the appropriate time via a coded command selected through user interface.

In variance stimulation, the tactile stimulation element 21, 121 is controlled to provide a series of stimuli in a sequence. The duration of the single stimulus, the duration of the pause between stimuli, or both may be customized to address different therapeutic purposes.

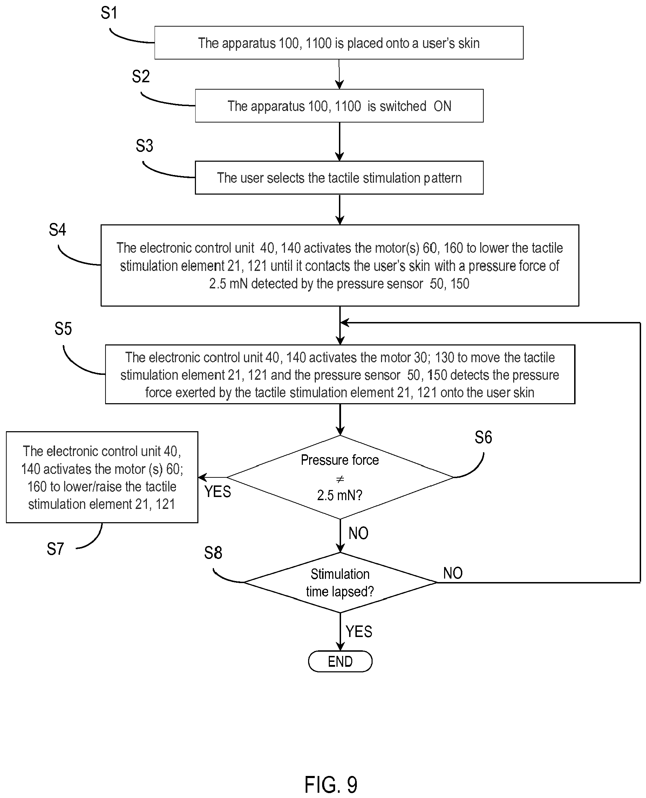

With reference to Figure 9, a method for tactile stimulation of C-Tactile afferents according to the present invention will now be described, the method being carried out by using the apparatus 100, 1100 described above and illustrated in Figures 1-8.

The method starts at step S1, in which the apparatus 100, 1100 is placed onto the user's skin, for example onto the area of the volar forearm, so that the tactile stimulation element 21, 121 is in contact with the user's skin. The step S1 is followed by a step S2, in which the apparatus 100, 1100 is switched ON by the user, and by a step S3, in which the user selects the tactile stimulation pattern.

At this point, the apparatus 100, 1100 starts to perform the tactile stimulation based on the selected tactile stimulation pattern.

In particular, at step S4, the electronic control unit 40, 140 activates the further motor(s) 60, 160 to adjust the position of the tactile stimulation element 21, 121 with respect to the user's skin 21, 121 until it contacts the user's skin and at the same time the pressure sensor 50, 150 detects the pressure force exerted by the tactile stimulation element 21, 121 onto the user's skin. In particular, the tactile stimulation element 21, 121 is lowered until it contacts the user's skin. When the pressure force exerted by the tactile stimulation element 21, 121 onto the user's skin reaches a value lower than 40 mN, preferably a value set at 2.5 mN, the method proceeds to step S5, in which the electronic control unit 40, 140 activates the motor 30, 130 to move, preferably rotate the tactile stimulation element 21, 121 at a controlled velocity, preferably at a velocity between 1 cm/s to 10 cm/s, with optimal velocity set to 3 cm/s, and at the same time the pressure sensor 50, 150 detects the pressure force exerted by the tactile stimulation element 21, 121 onto the user's skin. During movement, the tactile stimulation element 21, 121 stimulates each point of the user's skin substantially for less than 5 seconds and this advantageously avoids the stimulated C-Tactile afferents to exhibit a tendency to fatigue.

If the pressure force detected by the pressure sensor 50, 150 is different from 2.5 mN (step S6, YES), the electronic control unit 40, 140 activates (S7) the further motor(s) 60, 160 to adjust the position of the tactile stimulation element 21, 121 with respect to the user's skin, so that the pressure force exerted by the stimulation element 21, 121 onto the underlying user's skin becomes equal to about 2.5 mN. More specifically, and preferably, the tactile stimulation element 21, 121 is lowered/raised with respect to the user's skin. In particular, and as an example, if the apparatus is used for the tactile stimulation of the user's volar forearm, when the tactile stimulation element 21, 121 moves along the arm, due to the shape of arm itself, the skin in contact with the tactile stimulation element 21, 121 may become thinner, so as to move away from the tactile stimulation element 21, 121, or vice versa the skin may become thicker, so as to get closer to the tactile stimulation element 21, 121. In the first case, the pressure sensor 50, 150 detects that the pressure force exerted by the tactile stimulation element 21, 121 onto the underlying skin decreases with respect to the set value of about 2.5 mN and thus the tactile stimulation element 21, 121 is lowered, to restore the set pressure force to its optimal value. In the second case, the pressure sensor 50, 150 detects that the pressure force exerted by the tactile stimulation element 21, 121 onto the underlying skin increases with respect to the set value of about 2.5 mN, corresponding to the optimal pressure force value for CT stimulation, and thus the tactile stimulation element 21, 121 is raised, to restore the set pressure force to its optimal value.

The method proceeds for the desired stimulation time (step S8, NO) and terminates when the stimulation time is lapsed (step S8, YES).

Preferably, the method further comprises a step in which one or more biosensors connected to the user detect respective physiological data of the user and transmit the detected data to the electronic control unit 40, 140. The electronic control unit 40, 140, in turn, uses the received physiological data to regulate the parameters (e.g. time, frequency, and pattern of the stimulation) of tactile stimulation according to the user's physiological response.

Preferably, the method further comprises, between steps S2 and S3, a pressure force calibration procedure.

During this calibration step, the tactile stimulation element 21, 121 is lowered to contact the user's skin until the pressure sensor detects the correct pressure force value for CT stimulation and the electronic control unit 40, 140 stores the angular and vertical position of the tactile stimulation element 21, 121, along with the pressure data. The tactile stimulation element 21, 121 is then rotated of 15° or more degrees and the calibration procedure is repeated till reaching 360° degrees of rotation.

Subsequently, the electronic control unit 40, 140 utilizes the angular and vertical position and the pressure data stored therein, to calculate a morphological map of the user's skin to evaluate and compensate specific surface distortions (e.g., muscles, fat layers, body different morphological conformations).

From the above description, the features of the apparatus and of the method for tactile stimulation of the present invention, as well as the advantages thereof, are evident.

In particular, the inventive apparatus and the method allow to automatically regulate the tactile stimulation based on the physical characteristics of the user's skin, so as to stimulate the C-Tactile afferents with the above indicated optimal pressure force and velocity.

Further, due to its small size, the inventive apparatus is portable in different locations, such as hospitals, laboratories and the like, and may be also used in home treatments.

Moreover, due to its small size, a plurality of apparatuses may be used for simultaneously stimulating different body parts of a user. In this case all the apparatuses are connected to a personal computer or a remote server, preferably via wireless connections. In this configuration, the personal computer or the remote server can independently communicate with each apparatus to coordinate the stimulation upon large parts of the body. This method will allow to amplify the effect of the tactile stimulation, activating a large quantity of C-Tactile afferents at the same time, enhancing the therapeutic effect.

The person skilled in the art, in order to satisfy specific requirements, may make modifications to the embodiments of the apparatus for interoceptive tactile stimulation described above and/or replace parts described with equivalent parts, without departing from the scope of the attached claims. For example, although separate motors are used for controlling the movement and the lowering/raising of the stimulation element, a single motor may be used to control the stimulation element.

## Claims

1. An apparatus (100; 1100) for tactile stimulation of C-Tactile afferents comprising a casing (10; 110) configured to be positioned onto a user's skin and at least one tactile stimulation element (21; 121) carried by the casing (100; 110) and operated by a motor (30; 130) at a controlled velocity for generating at least one tactile stimulation;
**characterized in that** the apparatus (100; 1100) further comprises an electronic control unit (40; 140) and at least one pressure sensor (50; 150) in electrical communication with the electronic control unit (40; 140) to control the pressure force exerted by said at least one tactile stimulation element (21; 121) onto the user's skin by adjusting the position of the tactile stimulation element (21; 121) with respect to the user's skin, based on a feedback signal transmitted by said at least one pressure sensor (50; 150).

2. Apparatus (100; 1100) according to claim 1, further comprising a stimulation disk (20; 120) supported by the casing (10; 110), said at least one tactile stimulation element (21; 121) consisting of a tactile stimulation element (21; 121) projecting downwards from a peripheral portion of the stimulation disk (20; 120).

3. Apparatus (100) according to claim 2, wherein said at least one pressure sensor (50) is embedded in said at least one stimulation element (21).

4. Apparatus (1100) according to claim 2, wherein said at least one pressure sensor (150) is provided under the tactile stimulation element (121) so as to be interposed, in use, between the at least one tactile stimulation element (121) and the user's skin.

5. Apparatus (1100) according to claim 4, wherein said at least one pressure sensor (150) comprises a membrane (151) inside with a disk (152) made of graphene-composed polymer is housed, with said membrane (151) being connected to the casing (110) at a peripheral edge thereof.

6. Apparatus (100; 1100) according to any one of the preceding claims, wherein said at least one tactile stimulation element (21; 121) is controlled for adjusting the position of the tactile stimulation element (21; 121) with respect to the user's skin by at least one further motor (60, 160) in electrical connection with the electronic control unit (40, 140) to lower/raise the tactile stimulation element (21; 121) with respect to the user's skin.

7. Apparatus (100; 1100) according to any one of the preceding claims, wherein the at least one tactile stimulation element (21, 121) is controlled by the electronic control unit (40; 140) to move at a velocity between about 1 cm/s to about 10 cm/s, preferably at a velocity of about 3 cm/s.

8. Apparatus (100; 1100) according to any one of the preceding claims, wherein the at least one tactile stimulation element (21, 121) is controlled by the electronic control unit (40; 140) to produce a pressure force onto the user's skin lower than 40mN, and preferably equal to about 2.5 mN.

9. Apparatus (100; 1100) according to any one of the preceding claims, wherein the electronic control unit (40; 140) can be electrically connected with one or more biosensors (70; 80), which are configured to detect a physiological data of the user and transmit the detected data to the electronic control unit (40; 140), which, in turn, uses the received physiological data to regulate the tactile stimulation according to the user's physiological response.

10. Apparatus according to any one of the preceding claims for use in generating at least one adjustable tactile stimulation for generating pain analgesia and stress relief.

11. Method for generating at least one tactile stimulation of C-tactile afferents comprising the steps of:
- operating (S5) at least one tactile stimulation element (21; 121) at a controlled velocity to generate at least one tactile stimulation;
- adjusting (S7) the position of the at least one tactile stimulation element (21; 121) with respect to the user's skin to control the pressure force exerted by the at least one tactile stimulation element (21; 121), based on a feedback signal transmitted by at least one pressure sensor (50; 150).

12. Method according to claim 11, wherein the operating step (S4) comprises moving the at least one tactile stimulation element (21; 121) at a velocity between about 1 cm/s to about 10 cm/s, preferably at a velocity of about 3 cm/s.

13. Method according to claim 11 or 12, wherein the step of adjusting the position of the tactile stimulation element with respect to the user's skin comprises lowering/raising the at least one tactile stimulation element (21; 121) with respect to the user's skin to produce a pressure force onto the user's skin lower than 40mN, and preferably equal to about 2.5 mN.

14. Method according to any one of the preceding claims 11 to 13, further comprising upstream of said operating step (S5) a step of lowering (S4) the at least one stimulation element (21; 121) until it contacts the user's skin with a pressure force of about 2.5 mN detected by said at least one pressure sensor (50; 150).

15. Method according to any one of the preceding claims 11 to 14, further comprising a preliminary pressure force calibration procedure comprising the steps of:
- lowering the at least one tactile stimulation element (21; 121) to contact the user's skin until the at least one sensor pressure (50; 150) detects a pressure force value of about 2.5 mN;
- storing into the electronic control unit (40; 140) an angular and vertical position of the at least one tactile stimulation element (21; 121), along with pressure data;
- rotating the at least one tactile stimulation element (21; 121) of 15 or more degrees till reaching 360 degrees of rotation; and
- using the stored angular and vertical position and the pressure data to calculate a morphological map of the user's skin to evaluate and compensate specific surface distortions.
